# EUROPÄISCHE PATENTSCHRIFT

(11) **EP 4 756 419 B1**
(45) Veröffentlichungstag und Bekanntmachung des Hinweises auf die Patenterteilung: **07.10.2026**
(21) Anmeldenummer: 25214901.8
(22) Anmeldetag: 11.11.2025
(51) Int. Cl.: G01N 27/07, G01N 27/28, G01N 27/416, G01D 11/24, G01N 27/44, G01N 33/18

(54) **SENSORELEKTRODE FÜR DIE HALOGENBESTIMMUNG**
SENSOR ELECTRODE FOR HALOGEN DETERMINATION
ÉLECTRODE DE DÉTECTION POUR LA DÉTERMINATION D'HALOGÈNE

(30) Priorität: 04.12.2024 DE 102024136105
(43) Veröffentlichungstag der Anmeldung: 10.06.2026
(73) Patentinhaber: Analytik Jena GmbH+Co. KG, 07745 Jena (DE)
(72) Erfinder: Freundel, Marcel, 98693 Ilmenau (DE); Henneberg, Heiko, 99338 Plaue (DE); Traute, Henri-Alan, 98693 Ilmenau (DE); Kaufmann, Sophia, 07407 Rudolstadt OT Teichel (DE); Ehrling, Christiane, 98693 Ilmenau (DE)
(74) Vertreter: Endress + Hauser Group Services (Deutschland) AG+Co. KG

(56) Entgegenhaltungen:
- WO-A1-2013/000453
- CN-A- 102 884 402
- DE-A1- 10 359 885
- DE-A1- 102017 220 847
- DE-U1- 202011 101 241
- DE-U1- 202017 006 380
- US-A1- 2008 264 788

## Beschreibung

Die Erfindung betrifft eine Sensorelektrode für die Halogenbestimmung.

Im Stand der Technik ist es bekannt, für die Bestimmung von Spuren an Halogenen (außer Fluor, also Chlor, Brom oder Jod) die Biamperometrie einzusetzen. Dabei handelt es sich um eine Variante der Amperometrie, bei der zwei gleiche Arbeitselektroden zur Anwendung kommen. Ein Elektrolysestrom fließt nur dann, wenn an beiden Arbeitselektroden eine Stoffumsetzung stattfindet. Aus der Leitfähigkeit des Elektrolyten wird dann der Halogengehalt ermittelt. Ein typischer Anwendungsfall ist die AOX-Analytik gemäß ISO-Norm 9562. Dabei werden chlororganische Verbindungen an Aktivkohle adsorbiert. Die so vorbereiteten Proben werden in einem Ofen im Sauerstoffstrom verbrannt und das entstehende HCI (HX) wird nach der Schwefelsäuretrocknung in einer coulometrischen Messzelle quantifiziert.

Aus der DE 42 31 960 A1 lässt sich eine Sensorelektrode entnehmen, in der zwei Elektrodenstifte als Arbeitselektroden in einer Komponente zusammengeführt sind. In einem aus einer Keramik bestehenden Elektrodenschaft sind die elektrischen Verbindungskomponenten der Elektrodenstifte angeordnet. Die Elektrodenstifte überragen die Stirnseite des Elektrodenschafts oder sind bündig zur Stirnfläche, um für die Bestimmung des Chloridgehalts mit dem Elektrolyten in Kontakt zu kommen.

Bei einer solchen Sensorelektrode ist es für die Richtigkeit und Präzision der Messergebnisse wichtig, dass die wirksame Oberfläche der Elektrodenstifte und der Abstand zwischen ihnen bei allen Sensorelektroden stets gleich ist. Zudem ist darauf zu achten, dass die Elektrodenstifte in dem Keramikschaft gut abgedichtet sind. Beide Anforderungen führen dazu, dass eine Automatisierung der Herstellung der Sensorelektroden kaum möglich ist und auf kostenintensive Handarbeit zurückgegriffen werden muss.

Die DE 10 2008 054 659 A1 beschreibt, dass konduktive Leitfähigkeitssensoren mittels eines Spritzgießverfahrens erzeugt werden. Die DE 10 2018 121 787 A1 offenbart eine Elektrodenbaugruppe für einen amperometrischen Sensor, dessen Elektrodenkörper durch ein Spritzgussverfahren erzeugt wird. In der DE 10 2017 220 847 A1 wird ein Leitfähigkeitssensor beschrieben, der durch ein Kunststoffspritzgussverfahren erzeugt wird, wodurch die Elektroden eingebettet werden. Der DE 10 2021 112 181 A1 lässt sich ein Sensor entnehmen, dessen Leiterbahnen - z. B. aus Silber - umspritzt werden können. In der DE 10 2016 110 856 A1 wird angegeben, dass eine Elektrodenbaugruppe in Form eines spritzgegossenen Schalungsträgers hergestellt wird. Eine Elektrodenanordnung beschreibt auch die DE 20 2011 101 241 U1. Der DE 20 2017 006 380 U1 lässt sich ein Adapter für Kolloid-Elektroden entnehmen.

Eine Sensorelektrode für die Halogenbestimmung mit einer Leiterplatte als Einleger zeigt die CN 102 884 402 A.

Die Aufgabe der Erfindung besteht darin, eine Sensorelektrode für die Halogenbestimmung vorzuschlagen, die sich möglichst einfach fertigen lässt, ohne dass es zu Einbußen an der Qualität der Messungen kommt.

Die Aufgabe wird gelöst durch eine Sensorelektrode mit den Merkmalen des ersten Anspruchs.

Die erfindungsgemäße Sensorelektrode verfügt über mindestens zwei Elektrodenstifte, zwei längliche, plattenartige Einleger, eine Anschlusskomponente und einen Elektrodenschaft. Die Einleger sind länglich und plattenartig ausgestaltet. In einer Variante sind die Einleger zumindest teilweise aus Leiterplattenmaterial gefertigt. Die Einleger sind zum einen eine mechanische Grundstruktur der Elektrode und dienen zum anderen auch der elektrischen Kontaktierung der Elektrodenstifte.

Der Elektrodenschaft ist z. B. im Wesentlichen zylindrisch oder kreiszylindrisch ausgestaltet. Er verfügt über eine Stirnseite, aus der die Elektrodenstifte herausragen oder mit der sie bündig abschließen. Für die elektrischen Messungen sind die Elektrodenstifte mit den Einlegern verbunden, die sich in dem Elektrodenschaft befinden. Die Einleger sind durch den Elektrodenschaft geführt, werden durch den Schaft gehalten und sind dadurch auch gegenüber der Umwelt geschützt. Der Elektrodenschaft ist zumindest teilweise oder vollständig durch ein Spritzgussverfahren erzeugt. Das Spritzgussverfahren erlaubt es, Komponenten direkt in den Elektrodenschaft einzubetten und damit auch abzudichten. Die Einleger sind im Elektrodenschaft überdies sicher gegenüber Zug geschützt. Zudem können durch das Spritzgießen auch besondere Geometrien des Elektrodenschafts erzeugt werden. Weiterhin verfügt der Elektrodenschaft über eine weitere Stirnseite, die auch als Hals oder Hals-Stirnseite oder als Anschluss-Stirnseite bezeichnet werden kann und die von dem Prozess abgewandt ist und z. B. der Fixierung des Elektrodenschafts in einer Wandung usw. dient.

Insgesamt lassen sich kostengünstig Sensorelektroden mit reproduzierbaren Eigenschaften herstellen. Das Material für den Spritzguss ist vorzugsweise elektrisch isolierend. Vorzugsweise wird auch ein solches Material verwendet bzw. wird es derartig verarbeitet, dass der Elektrodenschaft eine möglichst glatte Außenfläche aufweist, die sich gut reinigen lässt und Verschleppungen minimiert. Indem die Elektrodenstifte teilweise in dem Elektrodenschaft eingebettet sind, ergibt sich eine gute Abdichtung, eine genaue Positionierung der Stifte sowie eine sehr gute mechanische Stabilität. Die Anschlusskomponente erlaubt die Verbindung der Elektrode mit Leitungen und daher auch mit weiteren Geräten.

Ein Elektrodenstift ist in einer Ausgestaltung als Kathodenstift ausgeführt und dient als Generatorkathode. Die Sensorelektrode wird vorzugsweise in einer coulometrischen Messzelle eingesetzt, die aus den beiden Sensorelektroden (vorzugsweise aus Silber) und einem Generatorelektrodenpaar besteht. Insbesondere im Falle der coulometrischen Chloridtitration besteht das Generatorsystem aus einer Platinkatode und einer Silberanode. Die Generatorkathode (vorzugsweise aus Platin) ist mit dem Kathodenstift in der Sensorelektrode integriert. Dies ist von Vorteil, da das Volumen der Messzelle klein ist und so die Elektroden platzsparend angeordnet werden können.

Bei einer Ausgestaltung der Sensorelektrode besteht der Elektrodenschaft zumindest teilweise aus einem Kunststoff. In dieser Ausgestaltung wird ein Spritzgussverfahren mit einem Kunststoff durchgeführt. Bei dem Kunststoff handelt es sich vorzugsweise um Polypropylen. Vorzugsweise ist der Kunststoff chemikalienbeständig, insbesondere gegenüber konzentrierter Essig- und Schwefelsäure. Beispielsweise handelt es sich bei dem Kunststoff um ein Polyvinylidenfluorid (PVDF), welches sich durch Härte, Dichtigkeit und Widerstandsfähigkeit gegenüber Chemikalien auszeichnet. Der Kunststoff zeichnet sich generell durch seine Spritzbarkeit aus. Es handelt sich also beispielsweise um Thermoplaste, Duroplaste oder Elastomere. Für die Auswahl des Kunststoffs ist seine Beständigkeit bedeutsam, die sich sowohl auf das Innere, also in Bezug auf die umspritzten Bauteilen (also vor allem thermische Beständigkeit), als auch auf das Äußere bei der Anwendung der Sensorelektrode in Bezug auf die ggf. vorhandenen Chemikalien (als vor allem chemische Beständigkeit) bezieht. Der Kunststoff sollte sich auch dadurch auszeichnen, dass er während der Anwendung der Sensorelektrode weder quillt noch schrumpft, um die Dichtheit zu gewährleisten.

Eine Ausgestaltung der Sensorelektrode beinhaltet, dass zwei Elektrodenstifte zumindest teilweise aus Silber bestehen. Die Sensorelektrode mit den Silber-Elektrodenstiften dient insbesondere der Bestimmung von Halogenen, vorzugsweise Chlorid. Durch das Spritzgussverfahren können die Elektrodenstifte dicht umschlossen werden. Dies im Gegensatz zu den Schwierigkeiten im Stand der Technik, der es beispielsweise nicht erlaubt, Silberstifte in Glas zu vergießen.

In einer weiteren Ausgestaltung bestehen zwei Elektrodenstifte zumindest teilweise aus Platin. Die zugehörige Sensorelektrode kann beispielsweise für die biamperometrische Bestimmung von SO2 verwendet werden.

Eine Ausgestaltung der Sensorelektrode beinhaltet, dass die Sensorelektrode ferner einen Kathodenstift aufweist. Dieser besteht vorzugsweise zumindest teilweise aus Platin.

Eine Ausgestaltung zeichnet sich dadurch aus, dass die zwei Einleger elektrische Leitungsstrukturen aufweisen, dass die zwei Elektrodenstifte, die zumindest teilweise aus Silber bestehen, mit den elektrischen Leitungsstrukturen des einen Einlegers verbunden sind, und dass der Elektrodenstift, der zumindest teilweise aus Platin bestehen, - mittelbar oder unmittelbar - mit den elektrischen Leitungsstrukturen des anderen Einlegers verbunden ist. In dieser Ausgestaltung werden die insgesamt drei Elektrodenstifte auf die zwei Einleger verteilt, indem zwei Silber-Elektrodenstifte mit den Leitungsstrukturen des einen Einlegers und ein Platin-Elektrodenstift mit den Leitungsstrukturen des anderen Einlegers verbunden werden. In einer der folgenden Ausgestaltungen ist insbesondere vorgesehen, dass der Platin-Elektrodenstift mittelbar über eine Adapterhülse mit den Leitungsstrukturen des anderen Einlegers verbunden ist.

In einer weiteren Ausgestaltung dient ein weiterer Elektrodenstift als Generatoranode. Diese Ausgestaltung ist vorzugweise damit verbunden, dass die Sensorelektrode über vier Elektrodenstift verfügt. Es ergibt sich ein kompaktes 4-Elektrodensystem, das als eine Baugruppe ausgeführt ist.

Eine Ausgestaltung der Sensorelektrode besteht darin, dass die Anschlusskomponente eine reversible elektrische Verbindung der Sensorelektrode mit einem Stecker erlaubt. In dieser Ausgestaltung kann die Sensorelektrode reversibel mit einem Stecker verbunden werden. Dies vereinfacht beispielsweise den Austausch oder die Reinigung der Sensorelektrode. Mit dem Stecker ist beispielsweise eine Steuer- oder Auswerteschaltung verbunden.

Eine Ausgestaltung der Sensorelektrode beinhaltet, dass der Elektrodenschaft ferner mindestens eine Aufnahmerille zum Aufnehmen einer Dichtkomponente aufweist. Bei der Dichtkomponente handelt es sich beispielsweise um einen O-Ring, der in die Aufnahmerille eingebracht wird. Die Dichtkomponente dichtet beispielsweise den Übergang zwischen dem Elektrodenschaft und einem Teil einer Messzelle ab, in der sich die zu vermessende Reaktionsflüssigkeit oder der Elektrolyt befindet.

Eine Ausgestaltung sieht vor, dass die Einleger so ausgestaltet und relativ zueinander angeordnet sind, dass Ebenen, in welchen die Einleger sich jeweils hauptsächlich erstrecken, im Wesentlichen senkrecht aufeinander stehen. In dieser Ausgestaltung sind zwei Einleger vorhanden, die sich jeweils hauptsächlich in Längsrichtung erstrecken und die z. B. jeweils teilweise als Leiterplatte oder aus Leiterplattenmaterial ausgestaltet sind. Die beiden Einleger sollen dabei senkrecht aufeinander stehen, sodass sie eine sichere Grundstruktur für den länglichen Elektrodenschaft bilden. Mindestens ein Einleger verfügt über eine elektrische Leitungsstruktur, mit der daher auch mindestens ein Elektrodenstift verbunden werden kann. In einer Ausgestaltung verfügen beide Einleger über jeweils eine Leitungsstruktur.

Die folgenden Ausgestaltungen beziehen sich auf die Einleger und wie sie relativ zueinander angeordnet sind bzw. welche geometrische Gesamtstruktur sie bilden. Zu bedenken ist dabei jeweils, dass die Einleger zum einen die Grundstruktur des Elektrodenschafts bilden und zum anderen der elektrischen Verbindung der Elektrodenstifte mit der Anschlusskomponente dienen. Letztendlich ergibt sich die äußere Erstreckung des Elektrodenschafts durch die Einleger. Die Geometrie der Einleger ist vorzugsweise so zu wählen, dass der bzw. die Einleger dem Spritzguss während der Fertigung des Elektrodenschafts standhalten müssen.

Eine Ausgestaltung beinhaltet, dass die Einleger so ausgestaltet und relativ zueinander angeordnet sind, dass die Einleger gemeinsam im Wesentlichen eine T-Form bilden. Die T-Form bezieht sich dabei auf den Großbuchstaben T. Dabei wird in einer Ausgestaltung der Querbalken leicht durch den Längsbalken durchstochen.

Eine Ausgestaltung sieht vor, dass die Einleger so ausgestaltet und relativ zueinander angeordnet sind, dass die Einleger gemeinsam im Wesentlichen eine Form des Plus-Zeichens bilden. In dieser Ausgestaltung bilden die zwei Einleger ein X-, Kreuz- oder PlusZeichen. **In** einer Ausgestaltung haben die vier Komponenten des Plus-Zeichens im Wesentlichen die gleiche Länge. Die Form der Einleger zeigt sich jeweils bei einem Schnitt senkrecht zu ihrer Längsachse

Es gibt also in einer Ausgestaltung eine mittlere Träger-Struktur, an dessen einem Ende sich eine weitere Quer-Teilstruktur anschließt (also eine T-Form). Alternativ kreuzen sich zwei Träger-Strukturen - vorzugsweise im Bereich ihrer jeweiligen Mitte - und bilden vorzugweise rechte Winkel miteinander (also eine Plus-Form). Alternativ zu der letztgenannten Variante können die zwei Träger-Strukturen sich unter einem stumpfen bzw. einem spitzen Winkel schneiden (also eine Kreuz- oder z. B. insbesondere Andreaskreuz-Form).

Erfindungsgemäß weist mindestens einer der zwei Einleger elektrische Leitungsstrukturen auf und sind die Einleger so ausgestaltet und relativ zueinander angeordnet, dass ein Einleger den anderen Einleger zumindest teilweise durchragt. Erfindungsgemäß werden die zwei Einleger miteinander verbunden, indem ein Einleger den anderen durchragt. Dadurch ergibt sich eine relativ einfach zu bewerkstelligende mechanische Grund-Stabilität. Die Erfindung geht insbesondere damit einher, dass ein Einleger derartig ausgestaltet ist, dass der andere Einleger durch ihn hindurchgeführt werden kann.

Eine ergänzende Ausgestaltung umfasst, dass die mindestens zwei Einleger über Lötpunkte miteinander verbunden sind. Die Lötpunkte dienen dabei auch der Versteifung der beiden Einleger. In einer Ausgestaltung sind Lötpunkte über die gesamte Strecke vorhanden, an denen die beiden Einleger in unmittelbarem Kontakt miteinander stehen.

In einer alternativen oder ergänzenden Ausgestaltung sind die mindestens zwei Einleger über Klebepunkte miteinander verbunden. In dieser Ausgestaltung wird somit eine Klebestoff verwendet.

Eine Ausgestaltung sieht vor, dass mindestens ein Elektrodenstift - und vorzugsweise zwei Elektrodenstifte - durch eine Lötverbindung mit den elektrischen Leitungsstrukturen des mindestens einen der zwei Einleger verbunden ist.

Eine Ausgestaltung beinhaltet, dass mindestens ein Elektrodenstift federnd in einer Adapterhülse angeordnet und elektrisch mit der Adapterhülse verbunden ist, und dass die Adapterhülse elektrisch mit den elektrischen Leitungsstrukturen des mindestens einen der zwei Einleger verbunden ist. Diese Ausgestaltung nimmt darauf Rücksicht, dass manche Arten von Elektrodenstiften sich nicht oder nicht ohne größeren Aufwand durch eine Lötverbindung mit anderen Komponenten - hier mit mindestens einem der zwei Einleger - verbinden lassen. Dies bezieht sich z. B. auf den Werkstoff Platin. Daher ist in der Ausgestaltung eine Adapterhülse vorgesehen, die zumindest eine mechanische Stabilisierung und eine grundlegende elektrische Kontaktierung ermöglicht. Die Adapterhülse ist dabei so ausgestaltet, dass sie z. B. eine Lötverbindung mit den Leitungsstrukturen des mindestens einen der zwei Einleger ermöglicht. Hierfür verfügt die Adapterhülse insbesondere über einen gefederten Kontakt, welcher den Elektrodenstift elektrisch kontaktiert.

Eine Ausgestaltung sieht vor, dass der in der Adapterhülse angeordnete Elektrodenstift in der Adapterhülse eingesteckt und infolge des Erzeugens des Elektrodenschafts durch das Spritzgussverfahren fixiert und abgedichtet ist. In dieser Ausgestaltung wird durch das Spritzgussverfahren, welches der Erzeugung des Elektrodenschafts dient, auch die Abdichtung des Bereichs des Elektrodenstifts und der Adapterhülse vorgenommen.

Eine Ausgestaltung beinhaltet, dass die Anschlusskomponente in Richtung der Einleger als mehrpolige Buchse ausgestaltet ist. In einer Ausgestaltung handelt es sich insbesondere um eine dreipolige Buchse, mit der drei Elektrodenstifte - mittelbar über die Einleger - verbunden sind.

Eine Ausgestaltung sieht vor, dass der Elektrodenschaft und die Anschlusskomponente so ausgestaltet und angeordnet sind, dass ein Querschnitt des Elektrodenschafts entlang des Elektrodenschafts im Wesentlichen stets symmetrisch ist. Die Einleger sind in dieser Ausgestaltung so ausgeführt und realisiert, dass der Querschnitt durch den Elektrodenschaft stets symmetrisch ist. Damit soll verhindert werden, dass es bei der Fertigung des Elektrodenschafts zu Materialanhäufungen kommt. Solche Anhäufungen führen oft zu Verzug und sind daher zu vermeiden. Insbesondere ist vorzugsweise dafür zu sorgen, dass die einzelnen Komponenten der Sensorelektrode innerhalb einer Form, welche ggf. bei dem Spritzgussverfahren verwendet wird, gleichbleibende Abstände zu der Innenfläche der Form haben. Damit ist es möglich, eine im Wesentlichen gerade und vollständig geschlossene Umhüllung der Innenteile des Elektrodenschafts zu erreichen. Überdies werden so während des Spritzgussverfahrens asymmetrisch wirkende Kräfte vermieden.

Weiterhin wird die Aufgabe gelöst durch ein Verfahren zur Herstellung einer Sensorelektrode für die Halogenbestimmung, wobei das Verfahren zumindest die folgenden Schritte umfasst: dass mehrere Elektrodenstifte relativ zueinander angeordnet werden, dass mindestens ein Elektrodenstift mit einem von mindestens zwei Einlegern elektrisch und mechanisch verbunden wird, dass um die Elektrodenstifte und um die zwei Einleger ein Elektrodenschaft zumindest teilweise mit einem Spritzgussverfahren so erzeugt wird, dass die Elektrodenstifte und die Einleger zumindest teilweise in dem Elektrodenschaft eingebettet sind und dass mindestens ein Elektrodenstift eine Stirnseite des Elektrodenschafts teilweise überragt, und dass nach dem Erzeugen des Elektrodenschafts bei mindestens dem einem die Stirnseite überragenden Elektrodenstift ein Maß, um welches er die Stirnseite überragt, verändert wird.

Die Ausführungen und Erläuterungen zur Sensorelektrode gelten entsprechend auch für das Verfahren und umgekehrt, sodass auf eine Wiederholung verzichtet wird. Bei der Sensorelektrode handelt es sich vorzugweise um eine mit dem Verfahren herstellte Elektrode.

Erfindungsgemäß werden die zwei Elektrodenstifte passend zueinander - z. B. in einer Form - angeordnet, um anschließend im Spritzgussverfahren den Elektrodenschaft zu erzeugen. Wenigstens ein Elektrodenstift wird mit einem der zwei Einleger verbunden und der Elektrodenschaft wird um die Stifte und die Einleger herum erzeugt. Die Elektrodenstifte stecken dabei mit einer Seite in dem Elektrodenschaft und ragen mit einer anderen Seite darüber hinaus oder schließen bündig mit der Oberfläche ab, um in Kontakt mit dem Medium zu kommen, dessen Chloridgehalt ermittelt werden soll. Weiterhin wird nach dem Erzeugen des Elektrodenschafts wenigstens ein Elektrodenstift so in den Abmessungen und damit in Bezug auf die Reaktionsfläche bearbeitet, dass sich z. B. eine gewünschte Messgenauigkeit ergibt.

In einer Ausgestaltung werden zwei Einleger ineinandergesteckt. Dann werden zwei Elektrodenstifte mit den Leitungsstrukturen mindestens eines Einlegers verlötet. Ein Elektrodenstift wird in eine Adapterhülse eingesteckt, die wiederum ebenfalls mit Leitungsstrukturen der Einleger elektrisch kontaktiert, z. B. verlötet ist. Dabei sind die elektrischen Verbindungen voneinander elektrisch isoliert. Die Fixierung der Anordnung geschieht in einem Werkzeug unter Ausnutzung der überstehenden Elemente, z. B. der Elektrodenstifte. Anschließend wird der Elektrodenschaft über das Spritzgussverfahren erzeugt.

Die Erfindung wird anhand der nachfolgenden Figuren näher erläutert.
- Fig. 1: zeigt einen Schnitt durch eine schematische Messanordnung für die Bestimmung des Chloridgehalts,
- Fig. 2: zeigt die Komponenten einer Sensorelektrode in Explosionsdarstellung,
- Fig. 3: zeigt einen Teil der Sensorelektrode der Fig. 2 im zusammengesetzten Zustand,
- Fig. 4: zeigt die Komponenten der Fig. 2 im montierten Zustand,
- Fig. 5: zeigt eine Variante der Komponenten einer Sensorelektrode im montierten Zustand und
- Fig. 6: zeigt eine räumliche Darstellung eines Schnitts durch die Komponenten der Fig. 5.

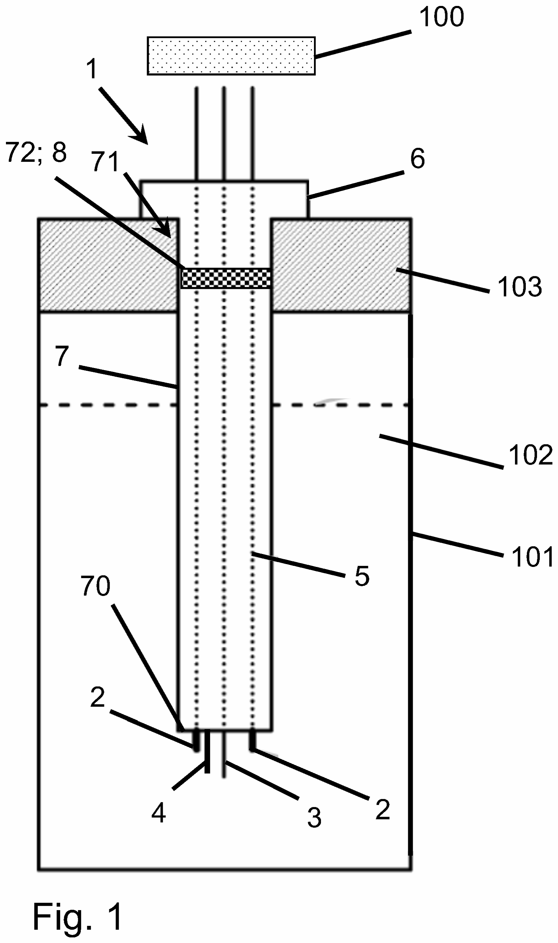

Die Fig. 1 zeigt eine Sensorelektrode 1, die sich in einer Messzelle 101 befindet. Die Messzelle 101 ist einseitig mit einem Messzellendeckel 103 verschlossen, wobei die Sensorelektrode 1 durch eine durchgehende Aussparung des Messzellendeckels 103 hindurchragt. Ein in einer Aufnahmerille 72 des Elektrodenschafts 7 angeordnete Dichtkomponente 8, die hier z. B. durch einen O-Ring gegeben ist, verschließt den Übergang zwischen dem Elektrodenschaft 7 und der Aussparung des Messzellendeckels 103.

In der Messzelle 101 befindet sich die Reaktionsflüssigkeit 102, in die die zwei gleich ausgeführten Elektrodenstifte 2, welche hier insbesondere aus Silber bestehen, hineinragen und für die Messung sorgen.

Der Elektrodenschaft 7 ist im Wesentlichen kreiszylindrisch ausgeführt und verfügt an seinem oberen Ende über eine Anschlusskomponente 6 mit einem größeren Außendurchmesser als der Innendurchmesser der Aussparung im Messzellendeckel 103. Dadurch wird der Elektrodenschaft 7 im Messzellendeckel 103 gehalten. Die Anschlusskomponente 6 ist weiterhin so ausgeführt, dass die Sensorelektrode 1 reversibel durch einen Stecker 100 elektrisch kontaktiert werden kann.

Gegenüber der Anschlusskomponente 6 sind die zwei Elektrodenstifte 2, die vorzugsweise aus Silber bestehen, in der Stirnseite 70 des Elektrodenschafts 7 angeordnet und dort eingebettet. Daher sind sie gut isoliert. Für die Messung ist weiterhin ein Elektrodenstift als Kathodenstift 3 vorhanden, der ebenfalls die Stirnseite 70 überragt, in der Stirnseite 70 eingebettet und zumindest mit einem Einleger 5 elektrisch kontaktiert ist. Der Kathodenstift 3 dient als Generatorkathode. Der vierte Elektrodenstift 4 erfüllt in der Messung die Aufgabe der Generatoranode. Der Einleger 5 sorgt für die elektrische Verbindungen zwischen den Elektrodenstiften 2, 3, 4 und der Anschlusskomponente 6 und bildet das geometrische Grundgerüst des vorzugsweise an sich kreiszylindrischen Elektrodenschafts 7.

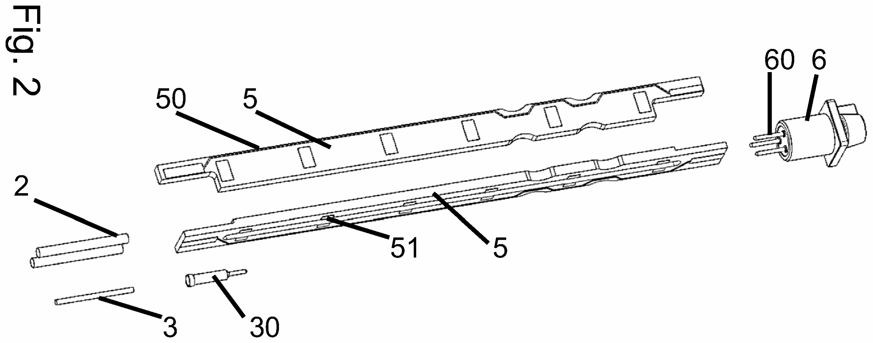

Die Fig. 2 zeigt eindrücklich, dass sich der Großteil der räumlichen Erstreckung der Sensorelektrode 1 aus der Form der hier zwei Einleger 5 ergibt. Die beiden Einleger 5 sind aus Leiterplattenmaterial ausgeführt und erstrecken sich hauptsächlich in Längsrichtung, also entlang der Längsachse der Sensorelektrode 1. Die Einleger 5 verfügen über Leitungsstrukturen 50, die der elektrischen Kontaktierung der Elektrodenstifte 2, 3 sowie der Anschlusskomponente 6 dienen.

Ein Einleger 5 verfügt über eine Aussparung 51, in die ein Teil des anderen Einlegers 5 eingebracht zu werden. Damit ergibt sich die mechanischen Grundstruktur des Elektrodenschafts 7.

An dem oberen (vgl. Fig. 1) bzw. hier rechten Ende befindet sich die Anschlusskomponente 6, die in der Anwendung der Sensorelektrode 1 mit einem Stecker 100 und daher mit einer für die Messung erforderlichen Gerätschaft verbunden werden kann. Die Anschlusskomponente 6 verfügt in dem Ausführungsbeispiel über drei Pole 60, die mit den drei Elektrodenstiften 2, 3 verbunden sind.

Für die Messungen sind die drei Elektrodenstifte 2, 3 vorhanden, von denen zwei Elektrodenstifte 2 aus Silber und ein Elektrodenstift 3 aus Platin besteht. Die Elektrodenstifte 2 aus Silber werden mit den Leitungsstrukturen 50 über Löten verbunden. Der Elektrodenstift 3 aus Platin wird in die Adapterhülse 30 eingebracht, um dort federnd gelagert und elektrisch kontaktiert zu sein. Die Abdichtung des Elektrodenstift 3 in der Adapterhülse 30 erfolgt - wie auch bei den anderen Elektrodenstiften 2 - über das Spritzgussverfahren, durch welches der Elektrodenschaft 7 erzeugt wird.

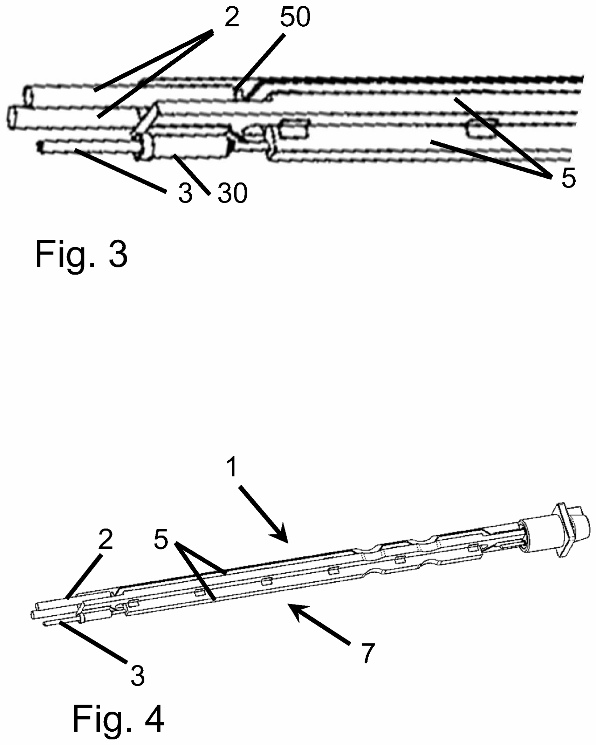

Die Fig. 3 zeigt das vordere Ende der Sensorelektrode 1 mit den drei Elektrodenstiften 2, 3. Die zwei Silber-Elektrodenstifte 2 sind direkt mit der Leitungsstruktur 50 des einen Einlegers 5 verbunden. Der Platin-Elektrodenstift 3 steckt in der Adapterhülse 30, die mit der Leitungsstruktur 50 des anderen Einlegers 5 elektrisch und mechanisch kontaktiert ist. Die Leitungsstruktur 50 kann dabei zumindest teilweise innerhalb oder auf dem jeweiligen Einleger 5 angeordnet sein.

Die Fig. 4 zeigt die zusammengesetzten Komponenten des zu umspritzenden Elektrodenschafts 7. Die Elektrodenstifte 2, 3 ragen stirnseitig hervor und erfahren durch den gespritzten Kunststoff ihre Abdichtung. Deutlich zeigt sich, dass die beiden Einleger sich einander durchragen und damit insgesamt senkrecht aufeinander stehen.

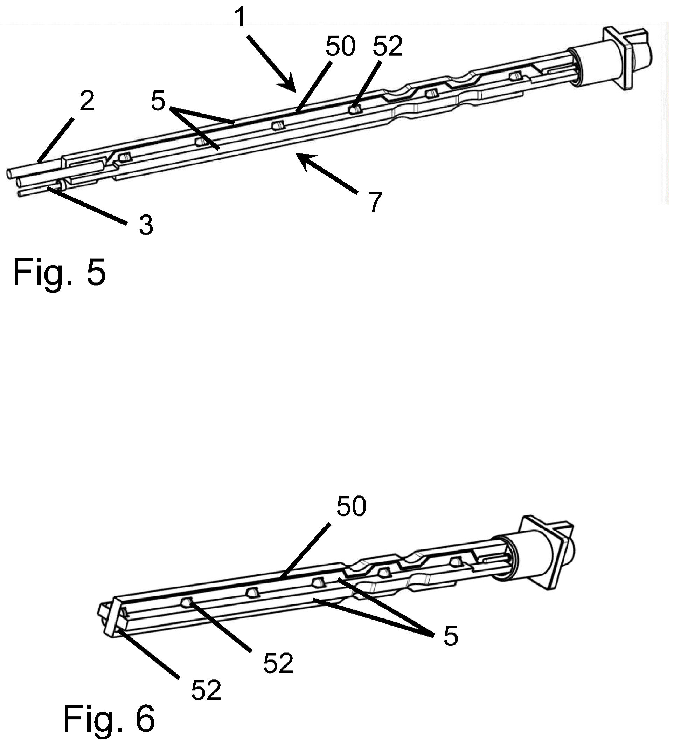

In der Fig. 5 ist gezeigt, wie sich in dem dargestellten Ausführungsbeispiel in dem Winkel zwischen den zwei Einlegern 5 insgesamt sechs Lötpunkte 52 in einer Reihe befinden. Die Lötpunkte 52 verbinden die beiden Einleger 5 miteinander und dienen der Versteifung der Anordnung aus den zwei Einlegern 5. Zu erkennen ist der Abstand zwischen den Lötpunkten 52 in der Spitze des von den zwei Einlegern 5 eingeschlossenen Winkels und der Leitungsstruktur 50 weiter außen auf dem Einleger 5. Dabei ist unterhalb des horizontal verlaufenden Einlegers 5 zu erkennen, dass mindestens eine weitere Reihe an Lötpunkten 52 vorhanden ist.

Die angeschnittene Darstellung der Fig. 6 zeigt, dass sich in den vier Schnittwinkeln zwischen den zwei Einlegern 5 und damit innerhalb der vier Ecken jeweils eine Reihe von Lötpunkten 52 befindet. In diesem Fall handelt es sich bei jeder Reihe um sechs Lötpunkte 52, sodass die beiden Einleger 5 insgesamt durch 24 Lötpunkte miteinander verbunden sind. Die Fig. 6 verdeutlicht dabei auch, wie der hier vertikal angeordnete Einleger 5 den hier horizontal angeordneten Einleger 5 mittig durchragt, sodass sich eine Kreuzform ergibt.

### Bezugszeichenliste

- 1: Sensorelektrode
- 2: Elektrodenstift
- 3: Elektrodenstift
- 4: Elektrodenstift
- 5: Einleger
- 6: Anschlusskomponente
- 7: Elektrodenschaft
- 8: Dichtkomponente
- 30: Adapterhülse
- 50: Leitungsstruktur
- 51: Aussparung
- 52: Lötpunkt
- 60: Pol der Anschlusskomponente
- 70: Stirnseite des Elektrodenschafts
- 71: Stirnseite des Elektrodenschafts
- 72: Aufnahmerille des Elektrodenschafts
- 100: Stecker
- 101: Messzelle
- 102: Reaktionsflüssigkeit
- 103: Messzellendeckel

## Patentansprüche

1. Sensorelektrode (1) für die Halogenbestimmung,
wobei die Sensorelektrode (1) mindestens zwei Elektrodenstifte (2, 3, 4), zwei längliche, plattenartige Einleger (5), eine Anschlusskomponente (6) und einen Elektrodenschaft (7) aufweist,
wobei der Elektrodenschaft (7) zwei einander gegenüberliegende Stirnseiten (70, 71) aufweist,
wobei die Elektrodenstifte (2, 3, 4) so angeordnet sind, dass die Elektrodenstifte (2, 3, 4) die eine Stirnseite (70) des Elektrodenschafts (7) überragen oder bündig mit der Stirnseite (70) abschließen,
wobei mindestens ein Elektrodenstift (2, 3, 4) mit mindestens einem der zwei Einleger (5) elektrisch und mechanisch verbunden ist,
wobei die zwei Einleger (5) in dem Elektrodenschaft (7) angeordnet sind,
wobei mindestens einer der zwei Einleger (5) elektrische Leitungsstrukturen (50) aufweist,
wobei die zwei Einleger (5) so ausgestaltet und relativ zueinander angeordnet sind, dass ein Einleger (5) den anderen Einleger (5) zumindest teilweise durchragt, wobei die Anschlusskomponente (6) an der anderen Stirnseite (71) des Elektrodenschafts (7) angeordnet ist,
wobei mindestens einer der zwei Einleger (5) mit der Anschlusskomponente (6) elektrisch und mechanisch verbunden ist,
wobei der Elektrodenschaft (7) zumindest teilweise durch ein Spritzgussverfahren erzeugt ist, und
wobei die Elektrodenstifte (2, 3, 4) zumindest teilweise in dem Elektrodenschaft (7) eingebettet sind.

2. Sensorelektrode (1) nach Anspruch 1,
wobei die zwei Einleger (5) elektrische Leitungsstrukturen (50) aufweisen, wobei zwei Elektrodenstifte (2) zumindest teilweise aus Silber bestehen,
wobei die Sensorelektrode (1) ferner einen zumindest teilweise aus Platin bestehenden Elektrodenstift (3) als Kathodenstift aufweist,
wobei die zwei Elektrodenstifte (2), die zumindest teilweise aus Silber bestehen, mit den elektrischen Leitungsstrukturen (50) des einen Einlegers (5) verbunden sind, und wobei der Elektrodenstift (3), der zumindest teilweise aus Platin bestehen, mit den elektrischen Leitungsstrukturen (50) des anderen Einlegers (5) verbunden ist.

3. Sensorelektrode (1) nach Anspruch 1 oder 2,
wobei ein weiterer Elektrodenstift (4) als Generatoranode dient.

4. Sensorelektrode (1) nach einem der Ansprüche 1 bis 3,
wobei die Anschlusskomponente (6) derartig ausgestaltet ist, dass die Anschlusskomponente (6) eine reversible elektrische Verbindung mit einem Stecker (100) erlaubt.

5. Sensorelektrode (1) nach einem der Ansprüche 1 bis 4,
wobei der Elektrodenschaft (7) ferner mindestens eine Aufnahmerille (72) zur Aufnahme einer Dichtkomponente (8) aufweist.

6. Sensorelektrode (1) nach einem der Ansprüche 1 bis 5,
wobei die Einleger (5) so ausgestaltet und relativ zueinander angeordnet sind, dass Ebenen, in welchen die Einleger (5) sich jeweils hauptsächlich erstrecken, im Wesentlichen senkrecht aufeinander stehen, und
wobei die Einleger (5) so ausgestaltet und relativ zueinander angeordnet sind, dass die Einleger (5) gemeinsam entweder eine T-Form bilden, indem ein Einleger (5) als Längsbalken den anderen Einleger (5) als Querbalken durchsticht, oder eine Form des Plus-Zeichens bilden.

7. Sensorelektrode (1) nach einem der Ansprüche 1 bis 5,
wobei die Einleger (5) so ausgestaltet und relativ zueinander angeordnet sind, dass die Einleger (5) gemeinsam im Wesentlichen eine Andreaskreuz-Form bilden.

8. Sensorelektrode (1) nach einem der Ansprüche 1 bis 7,
wobei die mindestens zwei Einleger (5) über Lötpunkte (52) miteinander verbunden sind.

9. Sensorelektrode (1) nach einem der Ansprüche 1 bis 8,
wobei mindestens ein Elektrodenstift (3) federnd in einer Adapterhülse (30) angeordnet und elektrisch mit der Adapterhülse (30) verbunden ist,
wobei die Adapterhülse (30) elektrisch mit den elektrischen Leitungsstrukturen (50) des mindestens einen der zwei Einleger (5) verbunden ist, und
wobei der in der Adapterhülse (30) angeordnete Elektrodenstift (3) in der Adapterhülse (30) eingesteckt und infolge des Erzeugens des Elektrodenschafts (7) durch das Spritzgussverfahren fixiert und abgedichtet ist.

10. Sensorelektrode (1) nach einem der Ansprüche 1 bis 9,
wobei der Elektrodenschaft (7) und die Anschlusskomponente (6) so ausgestaltet und angeordnet sind, dass ein Querschnitt des Elektrodenschafts (7) entlang des Elektrodenschafts (7) im Wesentlichen stets symmetrisch ist.

## Claims

1. Sensor electrode (1) for halogen determination,
wherein the sensor electrode (1) comprises at least two electrode pins (2, 3, 4), two elongated, plate-like inserts (5), a connection component (6), and an electrode shaft (7),
wherein the electrode shaft (7) has two opposing end faces (70, 71),
wherein the electrode pins (2, 3, 4) are arranged such that the electrode pins (2, 3, 4) protrude beyond one end face (70) of the electrode shaft (7) or are flush with the end face (70),
wherein at least one electrode pin (2, 3, 4) is electrically and mechanically connected to at least one of the two inserts (5),
wherein the two inserts (5) are arranged within the electrode shaft (7),
wherein at least one of the two inserts (5) comprises electrical conductive structures (50), wherein the two inserts (5) are designed and arranged relative to one another such that one insert (5) at least partially penetrates the other insert (5),
wherein the connection component (6) is arranged at the other end face (71) of the electrode shaft (7),
wherein at least one of the two inserts (5) is electrically and mechanically connected to the terminal component (6),
wherein the electrode shaft (7) is at least partially produced by an injection molding process, and
wherein the electrode pins (2, 3, 4) are at least partially embedded in the electrode shaft (7).

2. Sensor electrode (1) according to claim 1,
wherein the two inserts (5) comprise electrical conductor structures (50),
wherein two electrode pins (2) consist at least partially of silver,
wherein the sensor electrode (1) further comprises an electrode pin (3) consisting at least partially of platinum as a cathode pin,
wherein the two electrode pins (2), which consist at least partially of silver, are connected to the electrical conductive structures (50) of one insert (5), and
wherein the electrode pin (3), which consists at least partially of platinum, is connected to the electrical conductive structures (50) of the other insert (5).

3. Sensor electrode (1) according to claim 1 or 2,
wherein a further electrode pin (4) serves as a generator anode.

4. Sensor electrode (1) according to any one of claims 1 through 3,
wherein the connector component (6) is configured such that the connector component (6) allows for a reversible electrical connection with a plug (100).

5. Sensor electrode (1) according to any one of claims 1 through 4,
wherein the electrode shaft (7) further comprises at least one receiving groove (72) for receiving a sealing component (8).

6. A sensor electrode (1) according to any one of claims 1 through 5,
wherein the inserts (5) are configured and arranged relative to one another such that the planes in which the inserts (5) respectively extend primarily are substantially perpendicular to one another, and
wherein the inserts (5) are configured and arranged relative to one another such that the inserts (5) together form either a T-shape, with one insert (5) as a longitudinal bar piercing the other insert (5) as a transverse bar, or a plus sign shape.

7. Sensor electrode (1) according to any one of claims 1 through 5,
wherein the inserts (5) are configured and arranged relative to one another such that the inserts (5) together form, essentially, a St. Andrew's cross shape.

8. A sensor electrode (1) according to any one of claims 1 through 7,
wherein the at least two inserts (5) are connected to one another via solder joints (52).

9. A sensor electrode (1) according to any one of claims 1 through 8,
wherein at least one electrode pin (3) is resiliently mounted in an adapter sleeve (30) and is electrically connected to the adapter sleeve (30),
wherein the adapter sleeve (30) is electrically connected to the electrical conductive structures (50) of the at least one of the two inserts (5), and
wherein the electrode pin (3) arranged in the adapter sleeve (30) is inserted into the adapter sleeve (30) and is fixed and sealed as a result of the formation of the electrode shaft (7) by the injection molding process.

10. A sensor electrode (1) according to any one of claims 1 through 9,
wherein the electrode shaft (7) and the connection component (6) are configured and arranged such that a cross-section of the electrode shaft (7) along the length of the electrode shaft (7) is essentially always symmetrical.

## Revendications

1. Électrode de détection (1) destinée à la détermination des halogènes,
l'électrode de détection (1) comportant au moins deux tiges d'électrode (2, 3, 4), deux inserts allongés en forme de plaques (5), un composant de raccordement (6) et une tige d'électrode (7),
la tige d'électrode (7) comportant deux faces frontales opposées (70, 71),
les tiges d'électrode (2, 3, 4) sont disposées de telle sorte que les broches d'électrode (2, 3, 4) dépassent d'une face d'extrémité (70) de la tige d'électrode (7) ou affleurent ladite face d'extrémité (70),
au moins une broche d'électrode (2, 3, 4) est relié électriquement et mécaniquement à au moins l'un des deux inserts (5),
les deux inserts (5) étant disposés dans la tige d'électrode (7),
au moins l'un des deux inserts (5) comportant des structures conductrices électriques (50),
les deux inserts (5) sont conçus et disposés les uns par rapport aux autres de telle sorte qu'un insert (5) traverse au moins partiellement l'autre insert (5),
le composant de raccordement (6) étant disposé sur l'autre face frontale (71) de la tige d'électrode (7),
au moins l'un des deux inserts (5) est relié électriquement et mécaniquement au composant de raccordement (6),
la tige d'électrode (7) étant fabriquée au moins en partie par un procédé de moulage par injection, et
les broches d'électrode (2, 3, 4) étant au moins en partie noyées dans la tige d'électrode (7).

2. Électrode de capteur (1) selon la revendication 1,
dans laquelle les deux inserts (5) comportent des structures conductrices électriques (50),
dans laquelle deux broches d'électrode (2) sont constituées au moins en partie d'argent,
dans laquelle l'électrode de capteur (1) comporte en outre une broche d'électrode (3) constituée au moins en partie de platine, servant de broche cathodique,
dans laquelle les deux broches d'électrode (2), qui sont constitués au moins en partie d'argent, sont reliées aux structures de conduction électrique (50) de l'un des inserts (5), et
dans laquelle la tige d'électrode (3), qui est constituée au moins en partie de platine, est reliée aux structures de conduction électrique (50) de l'autre insert (5).

3. Électrode de capteur (1) selon la revendication 1 ou 2,
dans laquelle une autre tige d'électrode (4) sert d'anode de générateur.

4. Électrode de capteur (1) selon l'une des revendications 1 à 3,
dans laquelle le composant de raccordement (6) est conçu de telle sorte que le composant de raccordement (6) permette une connexion électrique réversible avec une fiche (100).

5. Électrode de capteur (1) selon l'une des revendications 1 à 4,
dans laquelle la tige d'électrode (7) comporte en outre au moins une rainure de logement (72) destinée à recevoir un composant d'étanchéité (8).

6. Électrode de capteur (1) selon l'une des revendications 1 à 5,
dans laquelle les inserts (5) sont conçus et disposés les uns par rapport aux autres de telle sorte que les plans dans lesquels les inserts (5) s'étendent principalement soient sensiblement perpendiculaires les uns aux autres, et
les inserts (5) étant conçus et disposés les uns par rapport aux autres de telle sorte que les inserts (5) forment ensemble soit une forme en T, un insert (5) en tant que barre longitudinale traversant l'autre insert (5) en tant que barre transversale, soit une forme de signe « plus ».

7. Électrode de capteur (1) selon l'une des revendications 1 à 5,
dans laquelle les éléments d'insertion (5) sont conçus et disposés les uns par rapport aux autres de telle sorte que les éléments d'insertion (5) forment ensemble essentiellement une croix de Saint-André.

8. Électrode de capteur (1) selon l'une des revendications 1 à 7,
dans laquelle les au moins deux éléments d'insertion (5) sont reliés entre eux par des points de soudure (52).

9. Électrode de capteur (1) selon l'une des revendications 1 à 8,
dans laquelle au moins une tige d'électrode (3) est disposée de manière élastique dans une douille d'adaptation (30) et est reliée électriquement à la douille d'adaptation (30),
la douille d'adaptation (30) étant reliée électriquement aux structures conductrices (50) de l'au moins une des deux inserts (5), et
la tige d'électrode (3) disposée dans la douille d'adaptation (30) étant insérée dans celle-ci et étant fixée et étanchée à la suite de la fabrication de la tige d'électrode (7) par le procédé de moulage par injection.

10. Électrode de capteur (1) selon l'une des revendications 1 à 9,
dans laquelle la tige d'électrode (7) et le composant de raccordement (6) sont conçus et agencés de telle sorte qu'une section transversale de la tige d'électrode (7) soit sensiblement toujours symétrique le long de la tige d'électrode (7).
